# EUROPÄISCHE PATENTSCHRIFT

(11) **EP 3 191 151 B1**
(45) Veröffentlichungstag und Bekanntmachung des Hinweises auf die Patenterteilung: **12.01.2022**
(21) Anmeldenummer: 15759646.1
(22) Anmeldetag: 08.09.2015
(51) Int. Cl.: A61M 1/36, A61M 5/44, H05B 3/00, A61M 1/16

(54) **HEIZVORRICHTUNG**
HEATING DEVICE
DISPOSITIF DE CHAUFFAGE

(30) Priorität: 12.09.2014 DE 102014013537
(43) Veröffentlichungstag der Anmeldung: 19.07.2017
(73) Patentinhaber: Fresenius Medical Care Deutschland GmbH, 61352 Bad Homburg (DE)
(72) Erfinder: PETERS, Arne, 61352 Bad Homburg (DE); ÖRTER, Gökhan, 35789 Weilmünster (DE)
(74) Vertreter: Herrmann, Uwe
(86) Internationale Anmeldenummer: PCT/EP2015/001811
(87) Internationale Veröffentlichungsnummer: WO 2016/037699

(56) Entgegenhaltungen:
- EP-A2- 2 468 390
- US-A- 3 657 517
- US-A- 6 069 343
- US-A1- 2009 299 273
- US-A1- 2010 145 273
- None

## Beschreibung

Die vorliegende Erfindung betrifft eine Heizvorrichtung für eine oder mehrere Komponenten eines extrakorporalen Kreislauf eines Blutbehandlungsgerätes, insbesondere eines Dialysators, Filters oder Adsorbers, wobei die Heizvorrichtung wenigstens einen Aufnahmebereich für die genannte Komponente sowie wenigstens ein Heizelement zur Beheizung der in der Aufnahme befindlichen Komponente aufweist.

Während einer extrakorporalen Blutbehandlung, wie beispielsweise während der Dialysebehandlung wird ein Teil des Blutes aus dem Körper in den extrakorporalen Kreislauf geleitet und dort beispielsweise durch Dialyse einem Reinigungsprozess unterzogen.

Da dabei eine übermäßige Abkühlung des Blutes bzw. des Patienten verhindert werden muss, ist es aus dem Stand der Technik bekannt, Heizeinrichtungen vorzusehen, die eine Beheizung einer Komponente des extrakorporalen Kreislaufes und damit auch eine Beheizung des darin befindlichen Blutes vornehmen.

Aus dem Stand der Technik sind beispielsweise Trommel-Schlauchheizer bekannt, bei denen ein Kunststoffschlauch mehrfach um eine beheizte Trommel gewickelt wird. Ein Nachteil derartiger Heizvorrichtungen besteht in einem großen Platzverbrauch, einem schwierigen Einlegen des Schlauches und in einem hohen Materialverbrauch.

Des Weiteren sind Mantel-Schlauchheizer bekannt, bei denen ein Kunststoffschlauch in einen beheizten Mantel eingebracht wird, was mit dem Nachteil eines vergleichsweise schwierigen Einlegens verbunden ist.

Eine weitere aus dem Stand der Technik bekannte Heizvorrichtung ist eine Folienheizung. Bei dieser Art der Beheizung wird eine Kunststofffolie an eine Heizfläche angekoppelt. Ein Nachteil bei einer derartigen Heizvorrichtung besteht in einem schwierigen Ankoppelvorgang sowie in einem hohen Platzverbrauch.

Die JP3028012 U offenbart einen Halter für einen Dialysator, der eine temperaturkontrollierbare Heizeinrichtung umfasst. Der Halter umfasst eine beheizbare Innenfläche mit einem Temperatursensor und kann mittels geeigneter Befestigungsmittel an einem Stativ befestigt werden.

Die US 2010 / 0 145 273 A1 offenbart eine Heizvorrichtung zum Erwärmen eines Infusionsschlauchs. Die Heizvorrichtung umfasst einen langgezogenen flexiblen Grundkörper, der über einen entlang seiner Längsausdehnung verlaufenden Schlitz zum Einbringen der zu erwärmenden Komponente verfügt. Darüber hinaus sind in dem flexiblen Körper Bohrungen vorgesehen, die zum Aufnehmen von Heizelementen dienen. Die Bohrungen verlaufen parallel zur Längsausdehnung des flexiblen Körpers.Ferner ist eine im Außenumfang des flexiblen Körpers angebrachte Beschichtung vorgesehen, die aus wärmeisolierendem Material besteht. Die Beschichtung ist derart ausgebildet, dass sie bei einem Einlegen der zu beheizenden Komponente flexibel bewegbar ist, um den Öffnungsspalt wahlweise zu vergrößern.

Die US 3 657 517 A zeigt ein lösbares Heizklemmband, das an eine Außenseite einer Flasche anklemmbar ist. Das Heizklemmband wird aufgeweitet und um die Außenseite eines Gefäßes geklemmt.

Aus dem Stand der Technik bekannte Anordnungen weisen zum Teil zwar ein gutes Handlung und eine gute Effizienz auf, sind jedoch mit dem Nachteil einer komplexen mechanischen Bauweise behaftet, wie beispielsweise bei der automatischen Ankopplung durch ein Gerät. Andere bekannte Ausführungen weisen zwar eine einfache mechanische Konstruktion, jedoch eine schlechte Effizienz, ein schwieriges Handling und einen großen Platzverbrauch auf, wie dies beispielsweise bei einer Schlauchtrommelheizung der Fall ist. Weitere bekannte Ausführungsformen, wie beispielsweise eine Folienkonstruktion in der Heizaufnahme mit händischer Folienankopplung bringen den Vorteil einer guten Effizienz mit sich, haben jedoch einen großen Platzverbrauch, erfordern ein schwieriges Handling und haben eine mechanisch komplexe Bauweise.

Der große Platzverbrauch bekannter Ausführungsformen resultiert daher, dass ein zusätzliches Element in das System eingebaut werden muss, das Flüssigkeit in einem Kunststoffartikel erwärmt.

Der vorliegenden Erfindung liegt die Aufgabe zugrunde, eine Heizvorrichtung der eingangs genannten Art dahingehend weiterzubilden, dass eine einfache Ankopplung der zu beheizenden Komponente sowie eine effiziente Beheizung möglich ist.

Diese Aufgabe wird durch eine Heizvorrichtung mit den Merkmalen des Anspruchs 1 gelöst. Danach ist vorgesehen, dass die Heizvorrichtung mehrere Schichten aufweist, wobei es sich bei wenigstens einer Schicht um eine flexible oder elastisch verformbare Schicht handelt. Durch eine derartige Schicht ist es möglich, dass sich die Heizvorrichtung auch bei Unebenheiten gut an die äußere Oberfläche der zu beheizenden Komponente anpasst bzw. dass die Ankopplung der Komponente an die Heizeinrichtung erleichtert ist, weil Toleranzen bei der Ankopplung durch die elastische bzw. flexible Schicht ausgeglichen werden können.

Vorzugsweise nutzt die vorliegende Erfindung bereits vorhandene Flächen und vereint damit eine platzsparende, effiziente Bauweise mit einer einfachen Konstruktion und einem einfachen Handling. Ein wesentlicher Vorteil der Erfindung besteht darin, dass die Heizvorrichtung gleichzeitig eine Halterung für einen Dialysator, Filter oder Adsorber darstellt und somit einen platz- und kostensparenden Effekt besitzt.

Erfindungsgemäß ist vorgesehen, dass die Heizvorrichtung wenigstens eine elastisch verformbare Schicht und wenigstens eine flexible Schicht aufweist.

Diese beiden Schichten liegen unmittelbar aneinander. Dabei kann die flexible Schicht die beheizbare Innenfläche der Aufnahme für die Komponente bilden.

Erfindungsgemäß ist vorgesehen, dass die Heizvorrichtung zumindest eine starre Schicht aufweist. Diese starre Schicht bildet die äußere Schicht der Heizvorrichtung, d.h. sie stellt deren Außenseite dar.

Vorzugsweise ist vorgesehen, dass die starre Schicht ein Elastizitätsmodul von 700 N/mm² oder mehr aufweist. Je höher das E-Modul ist, desto stabiler ist die Aufnahme.

Erfindungsgemäß ist vorgesehen, dass die Heizvorrichtung wenigstens eine starre Schicht, wenigstens eine elastisch verformbare Schicht und wenigstens eine flexible Schicht aufweist.

Die Kompressionskraft der elastisch verformbaren Schicht kann bei 25 % Kompression im Bereich von 0,01 N/mm² bis 0,7 N/mm² liegen. Je elastischer diese Schicht ist, desto vorteilhafter ist dies für die Heizvorrichtung. Wird die Geometrie des Dialysators, Filters oder Adsorbers in engen Toleranzen produziert, käme auch der Einsatz von Hartgummi oder einem Material mit einer vergleichbaren Elastizität in Betracht.

Die maximale Biegesteifigkeit der flexiblen Schicht bezogen auf die Breite der Folie kann bei 6,75 Nmm2/mm oder darunter liegen. Die flexible Schicht sollte eine möglichst geringe Biegesteifigkeit haben.

Die elastisch verformbare Schicht ist zwischen der starren Schicht und der flexiblen Schicht angeordnet.

Der erfindungsgemäße Schichtaufbau besteht darin, dass die Außenseite der Heizvorrichtung durch die starre Schicht gebildet wird, an diese die elastisch verformbare Schicht anschließt und die innere, d.h. die mit der Komponente in Kontakt stehende Schicht durch die flexible Schicht gebildet wird.

Um eine ausreichende Verformbarkeit zu gewährleisten, ist vorzugsweise vorgesehen, dass die elastisch verformbare Schicht vergleichsweise dick ausgeführt ist und vorzugsweise eine größere Dicke aufweist, als die starre Schicht und/oder als die flexible Schicht.

Die flexible Schicht kann beispielsweise Silikon, Polyimid, Karbon oder Aluminium, insbesondere Aluminiumfolie aufweisen oder aus einem oder mehreren dieser Komponenten bestehen.

Weiterhin kann die elastisch verformbare Schicht aus Gummi oder Schaum, insbesondere als Silikonschaum ausgeführt sein und eine oder beide dieser Komponenten aufweisen.

Die starre Schicht kann aus Kunststoff, insbesondere PP, PET, Aluminium oder Hartschaum bestehen oder einen oder mehrere dieser Komponenten aufweisen.

Die Wandung des Aufnahmebereichs, d.h. die Innenfläche der Heizvorrichtung, die mit der Komponente in Verbindung steht, kann durch die flexible Schicht oder durch die starre Schicht oder durch die elastische Schicht gebildet werden. Besonders vorteilhaft ist es, wenn die elastische und/oder die flexible Schicht die Innenseite der Aufnahme bildet, da sich dann ein gutes Anliegen der Heizvorrichtung an der Komponente erreichen lässt.

In weiterer Ausgestaltung der Erfindung ist vorgesehen, dass die elastische oder die flexible Schicht durch eine schwimmende Lagerung gebildet wird. In diesem Fall kann eine starre Schicht in einer schwimmenden Lagerung aufgenommen sein, wobei die starre Schicht beispielsweise die Aufnahme für die zu beheizende Komponente bildet. Bei einer solchen Ausgestaltung ergibt sich der Vorteil, dass Toleranzen bei der Ankopplung ausgeglichen werden, weil die Aufnahme nicht starr an dem Behandlungsgerät, insbesondere an einem Dialysegerät fixiert ist, sondern eine gewisse Beweglichkeit aufweist.

Die vorliegende Erfindung betrifft nicht nur die Heizvorrichtung als solche, sondern auch eine Kombination aus der Heizvorrichtung und einer in deren Aufnahmebereich angeordneten Komponente eines extrakorporalen Kreislaufes, bei der es sich insbesondere um einen Dialysator, einen Filter oder ein Schlauch handeln kann.

Um ein gutes Anliegen der Komponente an der beheizten Innenfläche der Heizvorrichtung sicherzustellen, kann vorgesehen sein, dass die Außenabmessung, wie insbesondere der Außendurchmesser der Komponente der Innenabmessung der Aufnahme entspricht oder diese übersteigt, bevor die Komponente in den Aufnahmebereich einlegt ist.

Das Heizelement kann beispielsweise zwischen zwei der genannten Schichten oder in wenigstens einer Schicht der Heizvorrichtung angeordnet sein. Denkbar ist es beispielsweise, dass es sich bei der flexiblen Schicht um das Heizelement handelt. Das Heizelement kann somit beispielsweise als Silikon-Heizelement ausgebildet sein.

Die vorliegende Erfindung betrifft des Weiteren ein Blutbehandlungsgerät, insbesondere ein Dialysegerät mit wenigstens einer Heizvorrichtung gemäß der Erfindung.

Die Heizvorrichtung weist vorzugsweise eine konkave Aufnahme auf.

Die Heizvorrichtung dient grundsätzlich vorzugsweise als Halterungselement für die zu beheizende Komponente.

Sie umgibt die zu beheizende Komponente vollumfänglich oder über einen Teilbereich des Umfangs, wie z.B. über einen Bereich von ca. 150° oder auch mehr. Vorzugsweise erfolgt eine Flächennutzung von > 50 %, d.h. vorzugsweise wird mehr als die Hälfte der beheizbaren Fläche der Komponente beheizt. Zur Rückseite, d.h. auf der von der Aufnahme abgewandten Seite ist die Heizvorrichtung vorzugsweise wärmeisoliert.

Denkbar ist es, dass die Heizvorrichtung aus mehreren Teilen besteht, die geöffnet werden können, um die Komponente einzulegen und sodann geschlossen werden. Das Verschließen kann dabei derart erfolgen, dass die Komponente in die Aufnahme gedrückt wird.

Bei der Komponente, die in der Aufnahme angeordnet wird bzw. sich darin befindet, kann es sich beispielsweise um eine Komponente mit starrer oder flexibler Außenfläche handeln, wie beispielsweise um einen Dialysator, einen Adsorber, einen Filter, wie z.B. einen Sterilfilter, einen Microfilter, um einen Schlauch etc.

Weitere Einzelheiten und Vorteile der Erfindung werden anhand eines in der Zeichnung beschriebenen Ausführungsbeispiels näher erläutert. Es zeigen.
- Figur 1:: eine Schnittansicht durch eine Heizvorrichtung gemäß der Erfindung und
- Figur 2:: eine weitere Schnittansicht durch eine Heizvorrichtung gemäß der Erfindung.

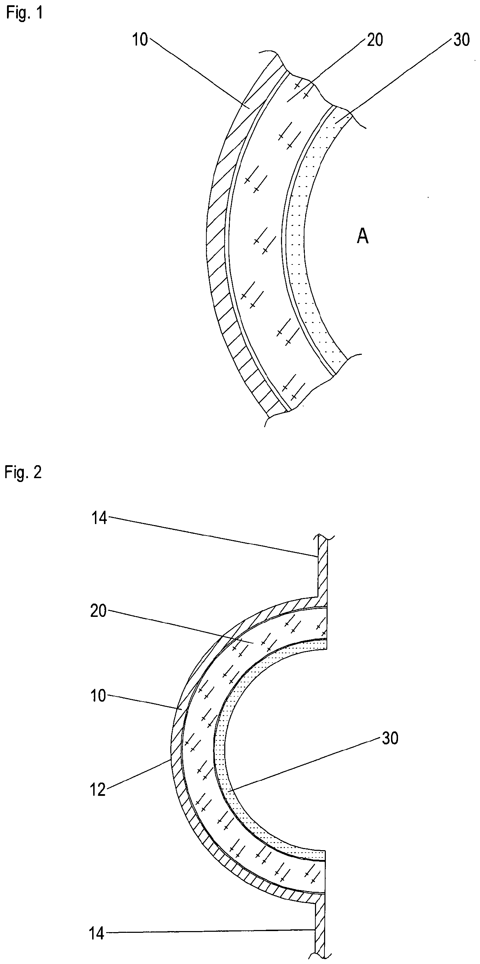

Figur 1 zeigt mit dem Bezugszeichen 10 die von dem Aufnahmebereich A abgewandte Außen- bzw. Rückseite der erfindungsgemäßen Heizvorrichtung, die beispielsweise aus einer Platte, wie z.B. aus einer Metallplatte oder aus Kunststoff, in einer schalengeometrischen Form besteht.

Der Aufnahmebereich A dient vorzugsweise zur Aufnahme eines zu beheizenden Disposables eines Blutbehandlungsgerätes, wie z.B. eines Filters oder eines Dialysators oder eines Adsorbers.

An diese starre Schicht 10 schließt sich eine elastisch verformbare Schicht 20 an, die z.B. aus einem Silikonschaum besteht. Die Schicht 20 kann beispielsweise eine Dicke im Bereich zwischen 5 mm und 15 mm und vorzugsweise eine Dicke von 10 mm aufweisen.

Auf diese Schicht folgt eine flexible innenliegende Schicht 30, die das Heizelement und die beheizte Innenseite der Heizvorrichtung bildet. Die flexible Schicht 30 besteht beispielsweise aus Silikon mit integrierten Heizdrähten oder einer beheizbaren Carbonfolie und weist einen geringeren Wärmewiderstand auf, als die elastische Schicht 20.

Zusätzlich können weitere flexible Schichten zur elektrischen Isolation oder mechanischen Oberflächenfestigkeit vorhanden sein.

Wie dies aus Figur 1 hervorgeht, sind die Schichten 10, 20, 30 in dem dargestellten Ausschnitt, d.h. auf Höhe des Aufnahmebereiches A zueinander konzentrisch angeordnet.

Aus Figur 1 geht weiter hervor, dass die Dicke der elastischen Schicht 20 größer ist als die Dicke der Schicht 10 sowie als die Dicke der Schicht 30.

Das starre Gehäuse 10 stellt die mechanische Stabilität der Heizvorrichtung her und drückt mittelbar über die elastisch verformbare Komponente 20 das flexible Element 30 an die in dem Aufnahmebereich A befindliche Komponente, wie beispielsweise an einen Dialysator. Durch die elastische Schicht 20 einerseits und durch die flexible Schicht 30 andererseits wird erreicht, dass sich das Heizelement - in Form der flexiblen Schicht 30 - gut an die Oberfläche und etwaige darin befindliche Unebenheiten anpassen kann, so dass ein besonders effizienter Wärmeübergang von der Heizvorrichtung zu der Komponente und somit letztlich auch eine besonders effiziente Erwärmung des in der Komponente strömenden Blutes erfolgen kann.

Figur 2 zeigt die gesamte Heizvorrichtung, von der ein Ausschnitt im Bereich des Aufnahmebereiches A in Figur 1 dargestellt ist.

Das Bezugszeichen 10 kennzeichnet das Gehäuse, das einen konkaven Abschnitt 12 aufweist, in dem sich die verformbare Schicht 20 und die flexible Schicht 30 befinden. An den konkaven Abschnitt schließen sich nach oben und unten gerade Abschnitte 14 an, die ohne die Schichten 20, 30 ausgebildet sind und die beispielsweise Befestigungsabschnitte bilden, mittels derer die Heizvorrichtung z.B. an einem Dialysegerät befestigt werden kann.

In der in den Figuren dargestellten Ausführungsform stellt die Heizvorrichtung eine Aufnahme dar, die beispielsweise den Dialysator hält, fixiert, sich anschmiegt und Toleranzen ausgleicht.

Die Heizvorrichtung besteht gemäß dem dargestellten Ausführungsbeispiel aus einem flexiblen, flachen Element zur Ankopplung an den Dialysator oder dergleichen und einer elastischen, dickeren Schicht. Das flexible, flache Element, das beispielsweise aus Silikon oder Polyimid oder Carbon oder Aluminiumfolie besteht, hat einen geringeren Wärmewiderstand als das elastische, verformbare dickere Element, das beispielsweise als Schaum, z.B. als Silikonschaum ausgeführt sein kann.

Ein starres Gehäuse verleiht der Heizvorrichtung die ausreichende mechanische Stabilität. Es kann beispielsweise aus PP oder PET oder auch aus einem Metall, wie z.B. Aluminium oder aus Hartschaum bestehen. Es drückt die verformbare Heizkomponente, d.h. beispielsweise die flexible Schicht an die zu beheizende Komponente, wie beispielsweise an die Oberfläche eines Dialysators, Filters etc.

Diese Komponente kann beispielsweise ein Kunststoffgehäuse mit geringer Wärmeleitung oder ein Gehäuse mit hoher Wärmeleitung, wie z.B. ein Gehäuse aus einem wärmeleitenden Kunststoff oder anderen Werkstoffe darstellen. Vorzugsweise ist das Gehäuse der Komponente, wie z.B. das Filter- oder Dialysatorgehäuse starr.

In einer weiteren Ausführungsform der Erfindung ist vorgesehen, dass die Heizvorrichtung aus einem starren Element, das zur Ankopplung an die zu beheizende Komponente dient, und aus einem elastischen Element aufgebaut ist. Das starre Element, das beispielsweise aus Aluminium oder Kunststoff ausgebildet sein kann, kann einen geringeren Wärmewiderstand als das elastische Element, das z.B. aus Gummi oder aus Schaumstoff bestehen kann, haben.

In einer weiteren Ausgestaltung der Erfindung ist die Aufnahme für die zu beheizende Komponente starr. Diese ist in ein einem elastischen bzw. flexiblen Element schwimmend gelagert. Dieses Element kann sich beispielsweise an dem Gehäuse und insbesondere an der Maschinenplatte eines Dialysegerätes befinden.

Durch die vorliegende Erfindung werden eine gute Ankopplung, ein einfaches Einlegen der zu beheizenden Komponente und eine effiziente Flächennutzung ermöglicht.

Vorzugsweise weist die Heizvorrichtung einen Temperaturfühler und eine Temperaturregeleinheit auf, die die Temperatur auf einem bestimmten Wert oder in einem bestimmten Bereich hält.

## Patentansprüche

1. Heizvorrichtung für eine oder mehrere Komponenten eines extrakorporalen Kreislaufes eines Blutbehandlungsgerätes, umfassend:
einen Aufnahmebereich (A) für die Komponente,
ein Heizelement zur Beheizung der in der Aufnahme befindlichen Komponente,
eine elastisch verformbare Schicht (20), und
eine flexible Schicht (30),
**dadurch gekennzeichnet, dass**
die Heizvorrichtung eine starre Schicht (10) aufweist, und
die elastisch verformbare Schicht (20) zwischen der starren Schicht (10) und der flexiblen Schicht (30) angeordnet ist, wobei
ein Schichtaufbau des Heizelements so ausgeführt ist, dass eine Außenseite der Heizvorrichtung durch die starre Schicht (10) gebildet ist, an die sich die elastisch verformbare Schicht (20) anschließt und die innere, mit der Komponente in Kontakt bringbare Schicht durch die flexible Schicht (30) gebildet ist.

2. Heizvorrichtung nach Anspruch 1, **dadurch gekennzeichnet, dass** die starre Schicht (10) ein Elastizitätsmodul von 700 N/mm² oder mehr aufweist.

3. Heizvorrichtung nach einem der vorhergehenden Ansprüche, dass die Heizvorrichtung wenigstens eine bzw. die genannte elastisch verformbare Schicht (20) aufweist, deren Kompressionskraft bei 25 % Kompression im Bereich von 0,01 N/mm² bis 0,7 N/mm² liegt.

4. Heizvorrichtung nach einem der vorhergehenden Ansprüche, **dadurch gekennzeichnet, dass** die flexible Schicht (30) eine maximale Biegesteifigkeit aufweist, die bezogen auf die Breite der Schicht bei 6,75 N mm²/mm oder darunter liegt.

5. Heizvorrichtung nach einem der vorhergehenden Ansprüche, **dadurch gekennzeichnet, dass** die elastisch verformbare Schicht (20) eine größere Dicke aufweist, als die starre Schicht (10) oder als die flexible Schicht (30).

6. Heizvorrichtung nach einem der vorhergehenden Ansprüche 1 - 4, **dadurch gekennzeichnet, dass** die elastisch verformbare Schicht (20) eine größere Dicke aufweist, als die starre Schicht (10) und als die flexible Schicht (30).

7. Heizvorrichtung nach einem der vorhergehenden Ansprüche, **dadurch gekennzeichnet, dass** die flexible Schicht (30) Silikon, Polyimid, Karbon oder Aluminium, aufweist oder aus einem oder mehreren dieser Komponenten besteht.

8. Heizvorrichtung nach einem der vorhergehenden Ansprüche, **dadurch gekennzeichnet, dass** die elastisch verformbare Schicht (20) aus Gummi oder Schaum, ausgeführt ist oder eine oder beide diesen Komponenten aufweist.

9. Heizvorrichtung nach einem der vorhergehenden Ansprüche, **dadurch gekennzeichnet, dass** die starre Schicht (10) aus Kunststoff, Aluminium oder Hartschaum besteht oder einen oder mehrere dieser Komponenten aufweist.

10. Heizvorrichtung nach einem der vorhergehenden Ansprüche, **dadurch gekennzeichnet, dass** die Komponente ein Dialysator, ein Filter oder ein Schlauch ist.

11. Heizvorrichtung nach Anspruch 10, **dadurch gekennzeichnet, dass** die Außenabmessung der Komponente der Innenabmessung der Aufnahme entspricht oder diese übersteigt.

12. Heizvorrichtung nach einem der vorhergehenden Ansprüche, **dadurch gekennzeichnet, dass** das Heizelement zwischen zwei der genannten Schichten oder in wenigstens einer Schicht der Heizvorrichtung angeordnet ist oder dass das Heizelement in der flexiblen Schicht angeordnet ist.

13. Heizvorrichtung nach einem der vorhergehenden Ansprüche 1 - 11, **dadurch gekennzeichnet, dass** das Heizelement zwischen zwei der genannten Schichten oder in wenigstens einer Schicht der Heizvorrichtung angeordnet ist und dass das Heizelement in der flexiblen Schicht angeordnet ist.

14. Blutbehandlungsgerät mit wenigstens einer Heizvorrichtung gemäß einem der Ansprüche 1 bis 13.

15. Gerät nach Anspruch 14, wobei das Blutbehandlungsgerät ein Dialysegerät ist.

## Claims

1. A heating device for one or more components of an extracorporeal circuit of
a blood treatment device comprising
a reception region (A) for the component;
a heating element for heating the component located in the receiver,;
an elastically deformable layer (20); and
a flexible layer (30),
**characterized in that**
the heating device has a rigid layer (10); and
the elastically deformable layer (20) is arranged between the rigid layer (10) and the flexible layer (30), with
a layer structure of the heating element being designed such that an outer side of the heating device is formed by the rigid layer (10) which the elastically deformable layer (20) adjoins and such thar the inner layer that can be brought into contact with the component is formed by the flexible layer (30).

2. A heating device in accordance with claim 1, **characterized in that** the rigid layer (10) has a Young's modulus of 700 N/mm² or more.

3. A heating device in accordance with one of the preceding claims, that the heating device has at least one or said elastically deformable layer (20) whose compressive force at 25% compression is in the range from 0.01 N/mm² to 0.7 N/mm².

4. A heating device in accordance with one of the preceding claims, **characterized in that** the flexible layer (30) has a maximum bending resistance of 6.75 N/mm²/mm or less with respect to the width of the layer.

5. A heating device in accordance with one of the preceding claims, **characterized in that** the elastically deformable layer (20) has a greater thickness than the rigid layer (10) or than the flexible layer (30).

6. A heating device in accordance with one of the preceding claims 1 - 4, **characterized in that** the elastically deformable layer (20) has a greater thickness than the rigid layer (10) and than the flexible layer (30).

7. A heating device in accordance with one of the preceding claims, **characterized in that** the flexible layer (30) comprises silicone, polyimide, carbon or aluminum or consists of one or more of these components.

8. A heating device in accordance with one of the preceding claims, **characterized in that** the elastically deformable layer (20) is formed from rubber or foam or comprises one or both of these components.

9. A heating device in accordance with one of the preceding claims, **characterized in that** the rigid layer (10) consists of plastic, aluminum or hard foam or comprises one or more of these components.

10. A heating device in accordance with one of the preceding claims, **characterized in that** the component is a dialyzer, a filter or a tube.

11. A heating device in accordance with claim 10, **characterized in that** the outer dimension of the component corresponds to the inner dimension of the receiver or exceeds it.

12. A heating device in accordance with one of the preceding claims, **characterized in that** the heating element is arranged between two of said layers or in at least one layer of the heating device; or **in that** the heating element is arranged in the flexible layer.

13. A heating device in accordance with one of the preceding claims 1 - 11, **characterized in that** the heating element is arranged between two of said layers or in at least one layer of the heating device; and **in that** the heating element is arranged in the flexible layer

14. A blood treatment device having at least one heating device in accordance with one of the claims 1 to 13,

15. A device in accordance with claim 14, wherein the blood treatment device is a dialysis machine.

## Revendications

1. Dispositif de chauffage pour un ou plusieurs composants d'un circuit extracorporel d'un appareil de traitement du sang, comprenant :
une zone de logement (A) pour le composant,
un élément de chauffage pour chauffer le composant se trouvant dans le logement,
une couche déformable de manière élastique (20), et
une couche flexible (30),
**caractérisé en ce que**
le dispositif de chauffage comporte une couche rigide (10), et
la couche déformable de manière élastique (20) est disposée entre la couche rigide (10) et la couche flexible (30), dans lequel
une structure en couches de l'élément de chauffage est réalisée de telle manière qu'un côté extérieur du dispositif de chauffage est formé par la couche rigide (10), à laquelle succède la couche déformable de manière élastique (20), et la couche intérieure, pouvant être amenée en contact avec le composant, est formée par la couche flexible (30).

2. Dispositif de chauffage selon la revendication 1, **caractérisé en ce que** la couche rigide (10) présente un module d'élasticité de 700 N/mm² ou plus.

3. Dispositif de chauffage selon l'une des revendications précédentes, en ce que le dispositif de chauffage comporte au moins une ou ladite couche déformable de manière élastique (20), dont la force de compression, à une compression de 25 %, est comprise entre 0,01 N/mm² et 0,7 N/mm².

4. Dispositif de chauffage selon l'une des revendications précédentes, **caractérisé en ce que** la couche flexible (30) présente une rigidité à la flexion maximale qui est de 6,75 N mm²/mm ou moins, par rapport à la largeur de la couche.

5. Dispositif de chauffage selon l'une des revendications précédentes, **caractérisé en ce que** la couche déformable de manière élastique (20) présente une épaisseur supérieure à la couche rigide (10) ou à la couche flexible (30).

6. Dispositif de chauffage selon l'une des revendications précédentes 1 à 4, **caractérisé en ce que** la couche déformable de manière élastique (20) présente une épaisseur supérieure à la couche rigide (10) et à la couche flexible (30).

7. Dispositif de chauffage selon l'une des revendications précédentes, **caractérisé en ce que** la couche flexible (30) comporte de la silicone, du polyimide, du carbone ou de l'aluminium ou est constituée d'un ou de plusieurs de ces composants.

8. Dispositif de chauffage selon l'une des revendications précédentes, **caractérisé en ce que** la couche déformable de manière élastique (20) est réalisée en caoutchouc ou en mousse ou comporte un de ces composants ou les deux.

9. Dispositif de chauffage selon l'une des revendications précédentes, **caractérisé en ce que** la couche rigide (10) est constituée de matière plastique, d'aluminium ou de mousse solide ou comporte un ou plusieurs de ces composants.

10. Dispositif de chauffage selon l'une des revendications précédentes, **caractérisé en ce que** le composant est un dialyseur, un filtre ou un tuyau flexible.

11. Dispositif de chauffage selon la revendication 10, **caractérisé en ce que** la dimension extérieure du composant correspond à la dimension intérieure du logement ou est supérieure à celle-ci.

12. Dispositif de chauffage selon l'une des revendications précédentes, **caractérisé en ce que** l'élément de chauffage est disposé entre deux desdites couches ou dans au moins une couche du dispositif de chauffage ou **en ce que** l'élément de chauffage est disposé dans la couche flexible.

13. Dispositif de chauffage selon l'une des revendications précédentes 1 à 11, **caractérisé en ce que** l'élément de chauffage est disposé entre deux desdites couches ou dans au moins une couche du dispositif de chauffage et **en ce que** l'élément de chauffage est disposé dans la couche flexible.

14. Appareil de traitement du sang comportant au moins un dispositif de chauffage selon l'une des revendications 1 à 13.

15. Appareil selon la revendication 14, dans lequel l'appareil de traitement du sang est un appareil de dialyse.
